# EUROPEAN PATENT SPECIFICATION

(11) **EP 1 562 568 B1**
(45) Date of publication and mention of the grant of the patent: **24.12.2008**
(21) Application number: 04778250.3
(22) Date of filing: 14.07.2004
(51) Int. Cl.: A61K 31/135, A61P 11/08

(54) **TREATMENT OF HEAVES**
BEHANDLUNG DER DÄMPFIGKEIT
TRAITEMENT DE LA POUSSE

(30) Priority: 24.07.2003 US 489863 P; 13.07.2004 US 889906
(43) Date of publication of application: 17.08.2005
(73) Proprietor: Courage Corporation Pty Ltd., Perth WA 6009 (AU)
(72) Inventor:
(74) Representative: Kirkham, Nicholas Andrew
(86) International application number: PCT/US2004/022645
(87) International publication number: WO 2005/009426

(56) References cited:
- WO-A-02/05630
- WO-A-91/09596
- WO-A-98/48810
- US-A- 5 362 755
- XIANG-YANG ZHANG ET ALL.: "Effects of eniantomers of beta-2-agonists on ACh release and smooth muscle contraction in the trachea" AMERICAN JOURNAL OF PHYSIOLOGY - LUNG CELLULAR AND MOLECULAR PHYSIOLOGY, vol. 274, no. 1, 1998, pages L32-L38, XP002333860
- DUVIVIER D H ET AL: "Aerosol therapy in the equine species" VETERINARY JOURNAL, BAILLIERE TINDALL, LONDON,, GB, vol. 154, no. 3, November 1997 (1997-11), pages 189-202, XP004762642 ISSN: 1090-0233

## Description

This application claims priority of provisional application Serial No. 60/489,863 filed on July 24, 2003, the disclosure of which is hereby incorporated by reference.

### BACKGROUND OF THE INVENTION

Heaves is a pulmonary disease in horses. Heaves is also observed in related species, as for example in mules and donkeys. Heaves often develops suddenly in horses and is a different disease than human chronic obstructive pulmonary disease (COPD) that develops slowly over time. Furthermore, heaves, but not COPD, has a strong atopic component which is due to allergic reactions to mold in hay. Heaves is characterized by airway smooth muscle constrictions, inflammation and bronchial mucus accumulation. It is believed that more than 75% of all horses in the USA suffer from heaves. Horses suffering from heaves are known to express bronchoconstriction during exercise and the usefulness of the animal will be substantially improved after administration of effective bronchodilating treatment. The disease called "heaves" varies in different areas of the world and in certain countries, as for example New Zealand, the disease may not even be called heaves, although the symptoms are very similar to those seen in American horses that suffer from heaves. The disease is called "equine inflammatory airway disease" or "allergic airway disease" or "small airway disease" in some countries. There may, in fact, be minor clinical differences between the symptoms of this disease in USA/Canada and in some other countries. It has been speculated that the differences in the expression of the symptoms may be due to different breeds of horses and also to different etiology, such as for example different types of mold causing atopic equine airways constriction.

The term "heaves" as used herein, encompasses the equine bronchoconstrictor disease, called heaves in the USA, and equine, heaves-like bronchoconstrictor syndromes, although the symptoms of the disease may be expressed differently in various animals, as stated above. Heaves in animal species related to horses, such as for example mules and donkeys, are encompassed in the present invention.

This invention relates to novel treatment of animals, particularly horses, suffering from heaves, using the optically pure R-enantiomer of the adrenergic beta-receptor agonist albuterol, having the chemical formula:

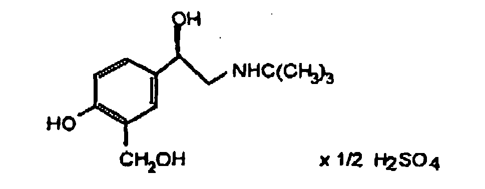

This invention also relates to the use of novel compositions containing the optically pure beta-receptor agonist R(-)-albuterol, intended for use in animals, particularly horses, suffering from heaves. The present invention offers potent, long-lasting therapeutic activity in animals suffering from heaves, while avoiding or reducing adverse effects including but not limited to pro-inflammatory activity, bronchial smooth muscle hyperreactivity, muscle tremor and tachycardia as well as avoiding or reducing the development of tolerance or hypersensitivity on repeated administration. The invention also provides a surprisingly improved oral absorption and duration of action of R(-)-albuterol in horses. This present invention relates to a use in methods of treating heaves, equine bronchospasms, exercise-induced bronchospasms and other ailments in horses with obstructive airway or allergic disorders, such as heaves-induced disturbance of normal sleep patterns, while unmasking anti-inflammatory activity of R(-)-albuterol, avoiding adverse effects residing in S(+)-albuterol, development of tolerance upon repeated dosing or, while dosed orally, the limited pattern of bronchial distribution that is obtained when bronchodilators are administered by inhalation.

The active compound of the present compositions and methods is an optical isomer of albuterol, which is described in U.S. Pat. No. 6,110,974, the disclosure of which is hereby incorporated by reference. Albuterol is called salbutamol outside the USA. Chemically, the R(-)-isomer of Albuterol as used herein refers to the optically pure (-) compound (R)-α¹-[[(1,1-dimethylethyl)amino] methyl]-4-hydroxy-1,3-benzene-dimethanol, sulfate (2:1) (salt), and to the corresponding base and any other biologically acceptable form or salt thereof. The racemic compound RS-albuterol is a well-known medication for asthma in human patients and is sold under various trade names, such as for example Proventil® (Schering) and Ventolin® (Glaxo). R(-)-albuterol is also an asthma medication, sold under the trade name Xopenex® (Sepracor) in the USA. The use of R(-)-albuterol to treat asthma in humans has been described by Barberich in USP 5,362,755. The use of R(-)-albuterol to inhibit premature contractions (tocolysis) of the pregnant uterus in humans have been described by Pesterfield in USP 5,708,036 and the use of R(-)-albuterol as a growth promoter in livestock was described by Aberg in USP 6,110,974.

Methods of making R(-)-albuterol have been described by Hamied in WO 02/48090 A1, by Gao in US patent 5,399,765 and by Ferrayoli et al. in Enantiomer, 2000, 5: 289-291. An online search revealed that racemic albuterol is included in more than 500 US patents. R(-)-albuterol is included in less than a dozen US patents.

Many organic compounds exist in optically active forms, i.e. they have the ability to rotate the plane of plane-polarized light. In describing an optically active compound; the prefixes R and S are used to denote the absolute configuration of the molecule about its chiral center(s). The prefixes (-) and (+) are employed to designate the sign of rotation of plane-polarized light by the compound, with (+) or 1 meaning that the compound is levorotatory. For a given chemical structure, a pair of enantiomers is identical except that they are non-superimposable mirror images of one another. A specific stereoisomer may also be referred to as an enantiomer, and a mixture of such isomers is often called an enantiomeric or racemic mixture.

Adrenergic drugs like albuterol exert their therapeutic effect through their action on the body's adrenergic beta-receptors. Albuterol has high affinity for adrenergic beta-2 receptors in the airways of animals and humans, which are activated by the drug. Albuterol is therefore called an "adrenergic beta-2 agonist". Since S-albuterol has less than 1% of the affinity of R-albuterol for adrenergic beta-receptors, it can be concluded that S-albuterol does not have any significant adrenergic beta-2 activity and the toxic effects of S-albuterol are non-adrenergic in nature. The very low affinity of S-albuterol to adrenergic beta-receptors is believed to be due to receptor affinity of optical impurities, consisting of R-albuterol, in test samples of S-albuterol. However, scientifically, the possibility of S-albuterol having very weak intrinsic affinity for adrenergic beta-receptors, cannot be excluded.

### CHEMISTRY

### R(-)-Albuterol Chemical Structure:

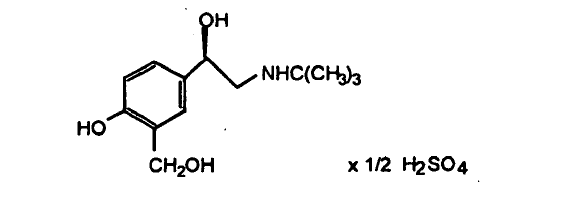

R(-)-Albuterol Molecular Formula: C₁₃H₂₁NO₃•1/2 H₂SO₄
R(-)-Albuterol Molecular weight: 288.31

### PHYSICAL AND CHEMICAL CHARACTERISTICS

R(-)-albuterol sulfate and R(-)-albuterol hydrochloride are white odorless, crystalline powders that are readily soluble in water. The chemical and optical purities of all batches used for biological studies have been >98%. The optical rotation of R(-)-albuterol sulfate, [α]²⁰d = -32 to -36 on dried basis. No polymorphs have been detected.

Based on their activity in humans, adrenergic beta-2 agonists are usually classified as "shortacting" or "longacting". Thus, albuterol and terbutaline are "shortacting beta-2 agonists", while salmeterol and formoterol are "long-acting beta-2 agonists".

Albuterol is usually administered by inhalation, e.g. using nebulizers or metered dose aerosol devices or it may be administered as inhaled powders to human patients, but the drug is known to undergo very rapid metabolism after oral administration and the much preferred route of administration is therefore by inhalation.

The S-isomer of albuterol has been found to cause significant side effects. Thus, adverse effects of S-albuterol and of racemic albuterol include but are not limited to non-adrenergic effects, such as, but not limited to (a) effects on the central nervous system (CNS-effects) such as for example nervousness, (b) cardiac effects such as for example cardiodepression, (c) effects on reproductive organs such as for example uterine hyperreactivity to oxytocin, (d) gastrointestinal side effects such as for example increased appetite, (e) respiratory side effects such as for example bronchial smooth muscle hyperactivity, bronchial smooth muscle hyperreactivity and pulmonary pro-inflammatory effects that are expressed as wheezing and coughing . In addition to the above, racemic albuterol may also cause (f) teratogenic side effects, which are believed to be associated with the S-isomer, since administration of the pure R-isomer of albuterol was not associated with teratogenic activity, when tested in animals (USP 5,708,036).

The symptoms of heaves include inflammation of the pulmonary airways of the horse. Inflammatory mediators that are released from pro-inflammatory cells cause the pulmonary inflammation in equine inflammation, as in many other forms of inflammation. Studies have been performed that demonstrate that S-albuterol, but not R-albuterol, is facilitating the release of inflammatory mediators from pro-inflammatory cells (Andersson et aL, ATS Internat Conf. Seattle 1995, Abstr.)

Animals have also been shown to develop tolerance to the bronchodilating effect of racemic albuterol. This is related to desensitization, which is one of the most clinically significant phenomena involving the beta-adrenergic receptor. This occurs after repeated administration of high concentrations of a receptor agonist. When the beta-receptor becomes desensitized to the agonist, higher doses (concentrations) of the agonist will be required to retain an equivalent physiological response. The problem of desensitization is especially significant in the repeated treatment of diseases involving bronchospasms, such as heaves, and is believed to be due to repeated exposure of the adrenergic beta-receptors to high concentrations of adrenergic beta-receptor agonists. Such high concentrations of the beta-receptor agonist at the receptor sites are more likely to occur during inhalation of the medication than during systemic (oral) drug administration. It is therefore of importance that R(-)-albuterol has now been found not to undergo intestinal or hepatic metabolism by M-form phenolsulfotransferase in the horse, but is well absorbed after oral drug administration to horses and has a long plasma half-life in horses. Thus, inhalation of R-albuterol is not necessary in horses and the development of tolerance can be avoided.

The term COPD stands for "Chronic Obstructive Pulmonary Disorder" and belongs to human medicine. The term is misleading when it refers to horses suffering from heaves, since heaves is not a "horse equivalent" to COPD in humans. Thus, heaves can develop quickly in horses while COPD is developing slowly and over time in humans. Heaves is believed to be caused by mold in hay when horses are kept in the barn during the winter months and heaves is not a major problem in countries where horses can be kept outside all year. The strong atopic component of heaves (allergy to mold from hay) is not present in human COPD. Thus, heaves is not the same disease as human COPD.

In the vast majority of cases, heaves is caused by an allergic reaction to mold spores found in the horse's environment. These spores are of respirable size and reach the small airways in the lungs. The clinical signs of heaves include three well-known components, bronchospasm, bronchial inflammation and excess mucus production. However, there may be additional symptoms of the disease that are less well known. The net effect of these processes is a narrowing of the airways and reduced capacity for airflow. Horses suffering from heaves are susceptible to various types of airways infections and to cough. It may therefore be advantageous to combine therapy with R-albuterol with medication that is directed towards pulmonary inflammatory events and towards pulmonary infections by various opportunistic microorganisms and with cough-suppressant drugs.

### SUMMARY OF THE INVENTION

It has now been discovered that the R-isomer of albuterol is a highly effective medication for heaves and for exercise-induced bronchoconstriction in animals, particularly in horses, and particularly for exercise-induced bronchoconstriction in horses suffering from heaves. The exact mechanism(s) of action for R-albuterol in restoring normal lung functions in the animal is/are not known, but anti-inflammatory and bronchodilating activities of R-albuterol while avoiding all pro-inflammatory effects and all the hyperreactivity of S-albuterol are believed to be crucial.

R-albuterol is known to be poorly and variably absorbed after oral administration because the drug is metabolized in the gastrointestinal tract and in the liver by an enzyme, called M-form phenolsulfotransferase (M-PST).

This enzyme, is known to stereoselectively metabolize R-albuterol (Hartman A.P. et al. Chirality 1998, 10:800-803). However, it has now unexpectedly been found that M-PST is not expressed in the horse. Thus, R-albuterol is not subject to presystemic metabolism in the intestines of the horse, but R-albuterol is well absorbed in the horse after oral administration. It was also found that R-albuterol has an unexpectedly long pharmacokinetic half-life in the horse after oral drug administration. Thus, chemical analyses of venous blood samples, taken from horses after oral administration of R-albuterol demonstrate good absorption and show that R-albuterol stays in the body of the horse for an unexpectedly long period of time after oral drug administration Furthermore, while R-albuterol can be inverted to S-albuterol in humans (Boulton et al. Clin Pharmacokinet., 2001, 40: 23-40), it has unexpectedly been found that no such inversion takes place in the horse.

Bronchial hyperreactivity by RS- and S-albuterol has previously been convincingly demonstrated only in isolated bronchial smooth muscle from guinea pigs. Studies in human patients have failed to convincingly confirm bronchial hyperreactivity by racemic albuterol (Boulton et al. Clin Pharmacokinet, 2001, 40: 23-40). It has now been found that S-albuterol, but not R-albuterol, is causing hyperreactivity in equine bronchial smooth muscle, which may explain the unexpectedly potent activity of the pure R-enantiomer of albuterol on equine bronchial smooth muscle.

### DETAILED DESCRIPTION OF THE INVENTION

The present invention relates to the uses of claims 1 to 10. These uses include eliciting therapeutic effects in animals suffering from heaves, which comprises administering to such animals an effective amount of R-albuterol, while avoiding the concomitant liability of adverse effects, increased toxicity, release of inflammatory mediators and development of bronchial hyperreactivity that are caused by S-albuterol. Furthermore, the development of tolerance, when administered by inhalation can be circumvented in horses, since it has now surprisingly been found that there is no presystemic metabolism of R-albuterol by M-PST in this species. Thus, advantageous therapeutic effects, including relief from exercise-induced bronchodilator effects in horses, suffering from bronchoconstriction and bronchial inflammation in heaves are achieved by utilizing the pure adrenergic beta-receptor agonist R(-)-albuterol while substantially limiting or completely avoiding the adverse effects, increased toxicity and pulmonary side effects of the corresponding distomer. The development of tolerance can be reduced or eliminated since R-albuterol, very surprisingly, is not subjected to presystemic metabolism in the intestines and the drug can therefore - and preferably - be administered orally to horses.

The present invention also encompasses use of a bronchodilator composition for the treatment of an animal, particularly a horse, in need of bronchodilating therapy which comprises the administration of an amount of R(-)-albuterol that is sufficient to alleviate bronchospasms but insufficient to cause adverse effects, increased toxicity, or development of tolerance. Bronchospasms can be observed in horses suffering from heaves, bronchitis and emphysema and bronchospasms can be induced in horses by exercise, particularly if the horses suffer from pre-existing pulmonary conditions such as for example heaves.

Since R(-)-albuterol has now been shown to be surprisingly well absorbed after oral administration, this route of administration is suitable for horses, suffering from heaves, since it is more convenient than the administration of inhalation therapy and since there is a limited pulmonary distribution after drug inhalation in horses suffering from heaves and other pulmonary conditions that encompass bronchoconstriction and/or airway mucus accumulation.

The sleep patterns of horses are vastly different from those of humans, but like humans, horses need a minimum amount of sleep, which can vary between horses and which can also change over time, young horses sleeping more than older horses. The sleep patterns of horses also varies with the weather, the season and with environmental factors. However, if the horse does not get the minimum sleep that he needs, he drifts off into what appears to be deep sleep while standing and he buckles at the knees. (Pascoe, E., Practical Horseman, September, 2000) It has now been observed that horses suffering from heaves have sleep patterns that are undesirably affected by their bronchial disorder and they will therefore not get the type and amount of sleep that meets their minimal requirements. The administration of R(-)-albuterol to a horse suffering from heaves, will normalize the sleep pattern and the horse will get the type and amount of sleep than meets his requirements.

Furthermore, although there is some variability from one patient horse to another, it is generally observed that, by administering an effective amount of the R-isomer of albuterol it is possible to accomplish a more "targeted" therapy. A more "targeted" therapy means that by using the single R-isomer, the compound's therapeutic activity can be taken advantage of without having consequences of the pharmacologic side effects of the S-isomer, which may occur upon administration of the racemic mixture.

The present invention provides a use for the treatment of equine inflammatory airways disease, in particular for effecting heaves and obtaining bronchodilatation. The invention may also provide prophylactic therapy in certain cases, as for example when R(-)-albuterol is administered to a horse to prevent exercise-induced bronchospasm. The teaching of the present invention is applicable in the therapy also of other equine inflammatory or obstructive airways diseases, in particular any such disease for which racemic adrenergic beta-receptor agonist drug therapy is commonly practiced, for example cystic fibrosis, emphysema and, chronic bronchitis, exercise-induced bronchospasm, asthma and, most especially, atopic bronchoconstriction in heaves.

The present invention avoids deleterious side effects hereinbefore described and frequently observed in horses consequent to conventional clinical usage of adrenergic beta-2 drugs as racemic mixtures. In particular the invention provides means to avoid, ameliorate or restrict deleterious side effects, e.g. systemic side effects such as increased systemic toxicity and side effects deleterious to the airways, such as bronchial constriction and pro-inflammatory effects. Due to the unexpectedly favorable metabolism of R-albuterol that has now been found in the horse, oral drug administration can be used, which will decrease the over-exposure of pulmonary beta-receptors to the drug after inhalation of the drug and therefore decrease or eliminate the development of tolerance to the drug.

Thus the invention provides means to prevent, avoid, ameliorate or restrict exacerbation of disease status, and will not inadvertently compromise normal lung function, or cause other side effects concomitant to conventional clinical usage of racemic beta-2 agonists, for example "anomalous", "rebound" or "paradoxical" bronchospasm and, especially, increase in airway obstruction, exacerbation of "late response" or non-specific bronchial reactivity or arterial hypoxemia.

Without limiting the present invention to any specific theory or mode of action, the present invention is in particular to be understood as providing a means for the avoidance, amelioration or restriction of exacerbation of airways smooth muscle constriction and/or of an inflammatory or other event associated with, or which is an etiological component of, inflammatory or obstructive airways disease in animals such as horses, e.g. heaves. Such events are to be understood as including for example, bronchial smooth muscle hyperactivity and hyperreactivity, release of inflammatory mediators from pro-inflammatory cells, inflammatory cell infiltration of the lungs or airways, connective tissue deposition or smooth muscle hyperplasia within the lungs or airways or other morphological changes associated with heaves.

The present invention also provides a means of preventing or reducing morbidity, e.g. heaves morbidity, ascribable to conventional, e.g. high dosage or long-term usage of racemic adrenergic beta-receptor agonist drugs.

The present invention is applicable in the therapy of bronchial constriction in horses of whatever type or genesis, such as for example allergic and non-allergic equine bronchoconstriction. It is in particular applicable to the treatment of allergic or atopic forms of heaves, as well as exercise-induced expression of bronchoconstriction.

Treatment of heaves is also to be understood as embracing treatment of horses suffering from symptoms associated with the cascade of pulmonary events following the release of pulmonary inflammatory mediators.

The term "adverse effects" includes but is not limited to muscle tremors, nervousness, increased heart rate, bronchoconstriction, dyspnea, coughing, excessive bronchial mucus formation, obvious chest pain, obvious chest discomfort, drying or irritation of the oropharynx and wheezing. Also included in the term "adverse effects" are fatigue, nausea, vomiting, sweating, muscle cramps, weakness and angina.

The term "substantially free of S-albuterol" as used herein means that the composition contains at least about 90% by weight of R(-)-albuterol and 10% or less by weight of S(+)-albuteroL In a more preferred embodiment the composition contains at least 98% by weight of R(-)-albuterol and 2% or less of S(+)-albuterol. In the most preferred embodiment the composition contains greater than 99% by weight of R(-)-albuterol and less than 1% by weight of S(+)-albuterol.

The term "eliciting a bronchodilator effect" means relief from the symptoms associated with obstructive airway diseases, which include but are not limited to symptoms like respiratory distress, wheezing, coughing, shortness of breath, or pressure in the chest and the like.

The term "development of tolerance" means that when administering the drug in repeated and high dosage or over a period of time, the amount of the drug given to the patient horse must be increased in order to achieve the same therapeutic effect as the lower dosage given at an earlier time.

The term "limited pattern of bronchial distribution when administered by inhalation" means that therapeutically efficacious quantities cannot penetrate into smaller bronchioles due to bronchoconstriction, inflammation or mucus accumulation in the airways.

Chemical synthetic methods of optically pure adrenergic beta-agonists are readily obtainable by methods known to those skilled in the art, e.g. by synthesis from an optically pure intermediate or resolution of the racemic compound into its isomers. Methods for resolution of racemic albuterol into the optically pure isomers have been described by Ferrayoli et al. Enantiomer, 2000, 5: 289-291, by Gao et aL in US Patent 5,399,765 and by Hamied et al. in WO 02/48090, the disclosures of which are hereby incorporated by reference.

The magnitude of a therapeutic dose of R(-)-albuterol in the management of disease will vary with the severity of the condition to be treated, and the dose of the drug. The dose of R(-)-albuterol used to treat horses with heaves will offer an amount sufficient to alleviate bronchospasms but insufficient to cause adverse effects. The dose needed to obtain an optimal therapeutic effect will vary and will depend on the dose frequency and will also vary according to the age, body weight, and response of the individual horse. In general, the total daily dose ranges when administered by inhalation, for the conditions described herein, is from about 1 microgram to about 200 micrograms per kilogram bodyweight two or four times daily. Preferably, a daily oral dose range should be between about 1 milligrams to 200 milligrams, two to four times daily; all doses will have to be titrated according to the severity of the symptoms as well known by the caring veterinary staff. A controlled-release tablet may be more convenient than an instant-release tablet and may contain approximately twice the amount of R(-)-albuterol as an instant release tablet or between 4 and 400 mg R(-)-albuterol, corresponding to 4.8 and 480 mg of R(-)-albuterol sulfate. Part of the dose of R(-)-albuterol in a controlled-release tablet may be contained in the coating of the tablet for immediate release and the remaining dose of R(-)-albuterol may be contained in the core of the tablet for release later. Controlled-release tablets may be given to the horse once or twice daily, while instant-release tablets may have to be given to the horse up to 4 times or more daily. In managing the horse suffering from heaves or from another bronchial ailment that includes bronchial smooth muscle constriction or hyperactivity, the therapy should be initiated at a lower dose, perhaps about twice daily dosing with 4 milligrams to about 12 milligrams and increased up to about twice daily dosage of 10 milligrams or higher depending on the horse's global response. It is further recommended that older horses and horses with impaired renal, or hepatic function, initially receive low doses, and that they be titrated based on individual response(s) and blood level(s). It may be necessary to use dosages outside these ranges as will be apparent to those skilled in the art. Further, it is noted that the treating veterinarian would know how and when to interrupt, adjust, or terminate therapy in conjunction with the individual horse's response. Analogous dosages and dosage forms apply to other animals.

The terms "an amount sufficient to alleviate bronchospasms but insufficient to cause adverse effects" are encompassed by the above-described dosage amounts and dose frequency schedule.

Any suitable route of administration may be employed for providing the horse with an effective dosage of R(-)-albuterol. For example oral, nasal, rectal, parenteral (subcutaneous, intramuscular, intravenous, etc.), transdermal, and like forms of administration may be employed. Dosage forms include tablets, troches, dispersions, suspensions, solutions, capsules, patches, and the like.

The pharmaceutical compositions of the present invention comprise R(-)-albuterol as the active ingredient, or a pharmaceutically acceptable salt thereof, and may also contain a pharmaceutically acceptable carrier, and optionally, other therapeutic ingredients.

The term "pharmaceutically acceptable salts" or "a pharmaceutically acceptable salt thereof refer to salts prepared from pharmaceutically acceptable non-toxic acids including inorganic acids and organic acids. Suitable pharmaceutically acceptable acid addition salts for the compound of the present invention include acetic, benzenesulfonic (besylate), benzoic, camphorsulfonic, citric, ethenesulfonic, fumaric, gluconic, glutamic, hydrobromic, hydrochloric, lactic, isethionic, maleic, malic, mandelic, methanesulfonic, mucic, nitric, pamoic, pantothenic, phosphoric, succinic, sulfuric, tartaric, p-toluenesulfonic, and the like. The sulfuric acid and the hydrochloric acid salts are particularly preferred.

The compositions of the present invention include compositions such as suspensions, solutions and elixirs; aerosols; mists; or carriers such as starches, sugars, microcrystalline cellulose, diluents, granulating agents, lubricants, binders, disintegrating agents, and the like. The compositions include compositions suitable for oral, rectal, parenteral (including subcutaneous, transdermal, intramuscular, and intravenous) and inhalation, although the most suitable route in any given case will depend on the condition being treated and the nature and severity of that condition. The most preferred routes of the present invention are: oral by tablets or capsules, inhalation and parenteral by a subcutaneous or intramuscular depot preparation. The doses of R(-)-albuterol may be conveniently presented in unit dosage form and prepared by any of the methods well known in the art of pharmacy.

In addition to the common dosage forms set out above, the compounds of the present invention may also be administered by controlled release means and/or delivery devices such as for example those described in U.S. Pat. Nos.: 3,845,770; 3,916,899; 3,536,809; 3,598,123; and 4,008,719, the disclosures of which are hereby incorporated by reference.

Several thousands of scientific publications describe various aspect of the preclinical and clinical pharmacology of racemic albuterol. An increasing number of publications deal with the in vitro and in vivo effects of R- and S-albuteroL

The invention is further defined by reference to the following examples describing in detail the pharmacological characterization of the compound, and the preparation of compositions of the present invention. It will be apparent to those skilled in the art, that many modifications, both to materials, and methods, may be practiced without departing from the purpose and interest of this invention.

### Example 1. Affinity to Adrenergic Beta-Receptors.

R(-)-albuterol is an adrenergic beta-receptor agonist with affinity to both adrenergic ß-1 and ß-2 receptors. As shown in the following table, R(-)-albuterol is twice as potent as the corresponding racemate, while the S(+)-isomer is practically inactive.

### Comparison of Affinities for Human ß-1 and ß-2 adrenergic receptors

| Compound | Kd for ß-2 receptors (nM) | Kd for ß-1 receptors (nM) |
|---|---|---|
| RS-albuterol | 668 | 2980 |
| R(-)-albuterol | 236 | 1540 |
| S(+)-albuterol | 33,600 | 111,000 |

### Conclusion:

R(-)-albuterol, but not S(+)-albuterol had affinity for the adrenergic beta-2 receptor.
It is believed that the very weak affinity for beta-receptors by S(+)-albuterol was due to optical impurities of R(-)-albuterol, since the test samples of S(+)-salbutamol used in these studies that had an optical purity ranging from 97% to >99%

### Example 2. Effects on cAMP Production

Stimulation of adrenergic ß-2 receptors result in an increased intracellular concentration of cyclic adenosine monophosphate (cAMP).
Results: The production of cAMP in human PC3 cells has been studied and the EC50-values for cAMP production were calculated to be 207nM for RS-albuterol, 100nM for R(-)-albuterol and 14,000nm for S(+)-albuterol.
Conclusion: R(-)-albuterol, but not S(+)-albuterol increased the production of intracellular cAMP.
The very weak activity of S(+)-albuterol in this study was probably due to optical impurities of R(-)-albuterol and/or to a weak intrinsic activity of the S(+)-isomer.

### Example 3. Effects on Airway Smooth Muscle Contractions

The following study was performed in vitro, using isolated guinea pig tracheas. The airway smooth muscle tissues were contracted by carbachol or ovalbumin or histamine. The inhibitory effects of R(-)- and S(+)-albuterol were calculated.

### Results: pD2 Values of the Enantiomers of Albuterol

| Contractile stimulus | S-albuterol | R-albuterol |
|---|---|---|
| Carbachol (10-6M) | 4.1 (2.5 5.7) | 7.1 (7.0-7.3) |
| Ovalbumin (10-5 g/ml) | 4.5 (1.9-7.2) | 8.3 (7.9-8.6) |
| Histamine (10-5M) | 5.3 (4.6-6.0) | 7.7 (7.5-7.9) |

Conclusions: R(-)-albuterol, but not S(+)-albuterol demonstrated potent airway smooth muscle relaxing activity. The very weak activity of S(+)-albuterol in this study was probably due to optical impurities of R(-)-albuterol and/or to a weak intrinsic activity of S(+)-albuterol

Example 4, Hyperreactivity by S-albuterol in equine tracheal smooth muscle. In this study, segments of equine bronchial smooth muscle are mounted on special holders and immersed in a physiologic solution containing glucose and aerated with 95% O2 and 5% CO2 at 37°C.. Isometric tension is measured with force transducers and registered electronically. The effects of R-, RS- and S-albuterol on carbachol-induced contractions are studied.
Results: All results obtained to date demonstrate that S-albuterol is causing bronchial hyperreactivity of equine bronchial smooth muscle, while R-albuterol is causing bronchorelaxation.

### Example 5. Anti-inflammatory Effects: Inhibition of Mediator Release

In this study, histamine was the marker compound for inflammatory mediators that are released from pro-inflammatory cells. The inhibition of stimulated histamine release from isolated human leukocytes (buffy coat) by test articles was studied.
The method is a modification of the methodology described by Nolte et al. (1988). Leukocytes were obtained from healthy volunteers and inflammatory mediator release was induced by incubation (20 min/37°C) with the Ca-ionophore A23187 (5 µM) in the presence or absence of a test article. Histamine was analyzed by enzyme-immuno assays (E.I.A.), using commercially available kits and a microplate reader (MRX, Dynatech). The test articles were tested in each assay at five concentrations ranging from 4×10⁻⁴ M to 10⁻⁶ M, in duplicate. In the same experiment, a reference compound was tested in duplicate at eight concentrations to obtain an inhibition curve to validate the experiment (not shown here).

Results: Neither R(-)-albuterol nor S(+)-albuterol inhibited the release of the marker inflammatory mediator in this study. However, S(+)-albuterol significantly and dose-dependently increased the release of the marker inflammatory mediator histamine from the leukocytes at concentrations of 10µM and higher.
Conclusions: Neither R(-)-albuterol nor S(+)-albuterol demonstrated anti-inflammatory activity by inhibition of the release of inflammatory mediators in this study. However, S(+)-albuterol demonstrated pro-inflammatory effects in this study.

### Example 6. Anti-inflammatory Effects: Inhibition of T-Cell Proliferation

Method: Antigen-specific T-cell lines were generated in the presence of recombinant human IL-2 and tetanus with or without varying concentrations of R(-)-albuterol and S(+)-albuterol alone or in combination. Parallel lines were generated in the presence of propranolol. Cells were evaluated for proliferation, apoptosis, and cytokine secretion.
Results: R(-)-albuterol significantly inhibited T-cell proliferation (77.0% ± 9.7% of control at 10(-8) mol/L and 61.1% ± 9.0% at 10(-7) mol/L). No influence was observed with S(+)-albuterol alone. However, the addition of S(+)-albuterol to R(-)-albuterol mediated a dose-dependent increase in proliferation. At equivalent concentrations of the two isomers, proliferation was unchanged from the control, whereas at 10(-6) mol/L of S(+)-albuterol, proliferation was enhanced. Both the inhibitory effects of R(-)-albuterol alone and the stimulating influence of R(-)- plus S(+)-albuterol were blocked in the additional presence of propranolol. R(-)-albuterol at 10(-8) mol/L inhibited IL-2 and IFN-gamma production. Racemic albuterol (10(-8) mol/L each) had no influence on cytokine production; however, the combination of 10(-8) mol/L R(-)-albuterol with 10(-6) mol/L of S(+)-albuterol stimulated production of IL-2 and IL-13. No effects were observed on apoptosis or cell viability.
Conclusions: The studies confirm the beta-adrenergic receptor-specific anti-inflammatory effects of R(-)-albuterol. The racemate had minimal influences on proliferation or cytokine production. The presence of excess S(+)-albuterol resulted in pro-inflammatory influences.

### Example 7. Metabolic studies.

Methods: The livers from recently slaughtered horses are homogenized and the cytosol is prepared by differential centrifugation. The protein concentration of the cytosolic preparation is determined by the Lowry method (Lowry O. H. et al.: Protein measurement with the foline phenol reagent. J. Biol. Chem. 1951, 193: 265-275). Cytosol (2 mg/ml protein) is assayed for sulfotransferase activity by incubating at 37□C for 50 minutes with adrenergic beta-2 agonist R(-)-albuterol and 3'-phosphoadenosine-5'-phosphosulfate (0.1 mM) in the presence of 50 mM phosphate buffer (pH 7.4), and 5 mM MgCl₂. Cytosol from the livers is incubated at 15□C. After removal of unmetabolized beta-2 agonist with ethyl acetate, free drug is released from the conjugate using sulfatase. The concentrations of beta-2 agonist enantiomers are analyzed by chiral HPLC assay.
Results: Test results obtained to date surprisingly demonstrate that the horse liver does not express M-PST. Results from tests with intestinal mucosa yielded the same surprising results.

### Example 8. Effects on Respiration in Horses

Methods: Respiratory mechanics is based on general principles of physics, where the pressure required to move an object is based on the mass of the object, the speed of the displacement, the resistance of the system, etc. Air is moved through the airways down to the lung by changes in pressure in the thorax, caused by displacement of the thoracic walls resulting from the movement of the respiratory muscles. During inspiration the thoracic cavity expands, causing a negative pressure in the thorax that attracts air inwards. During expiration, the thoracic cavity contracts, resulting in outward movement of air.

The technique to measure respiratory mechanics requires the determination of the intrathoracic pressure and the flow of air in and out the respiratory system. A tube positioned in the esophagus of the horse to measure the pressure within the thorax and a pressure transducer is placed at the end of the tube. The tube is small and its placement is well tolerated by horses. A pneumotachograph is placed on a facial mask and measures the flow of air. These two signals allow the calculation of various respiratory parameters such as tidal volume (quantity of air inspired during a breath), minute ventilation (volume of air inhaled per minute), airway resistance (horses heaves have an obstruction of the airway which impairs airflow), airway compliance (measures the elasticity of the lung; also abnormal in horses with heaves) and inertance. The calculation of inertance provide information on the elasticity of the lung.

### Example 9. Formulations

### ORAL FORMULATIONS

We have found that the horse unexpectedly does not express the enzyme M-form phenolsulfotransferase (M-PST) that is the cause of the poor oral availability and the short duration of R-albuterol in humans (Example 7, above). This finding makes it possible to design oral formulations of R(-)-albuterol that are not metabolized in the gut or in the liver by said enzyme, but are well absorbed and are offering long duration of therapeutic activity.

### Tablets:

| | Quantity per Tablet (mg.) | | |
|---|---|---|---|
| Formula | A | B | C |
| R(-)-albuterol | 0.1 | 1.00 | 4.00 |
| Lactose | 41.4 | 40.5 | 37.50 |
| Cornstarch | 3.0 | 3.0 | 3.0 |
| Water (per thousand Tablets)* | 30.0 ml | 30.0 ml | 30.0 ml |
| Cornstarch | 5.00 | 5.00 | 5.00 |
| Magnesium Stearate | 0.50 | 0.50 | 0.50 |
| Total weight | 50.00 | 50.00 | 50.00 |

| | | | |
|---|---|---|---|
| *The water evaporates during manufactured | | | |

The API (Active Pharmaceutical Ingredient; R-albuterol) is blended with the lactose until a uniform blend is formed. The smaller quantity of cornstarch is blended with the water to form the resulting cornstarch paste. This is then mixed with said uniform blend until a uniform wet mass is formed. The remaining cornstarch is added to the resulting wet mass and mixed until uniform granules are obtained. The granules are then screened through a suitable milling machine, using a 1/4 inch stainless steel screen. The milled granules are then dried in a suitable drying oven until the desired moisture content is obtained. The dried granules are then milled through a suitable milling machine, using 1/4 mesh stainless steel screen. The magnesium stearate is then blended and the resulting mixture is compressed into tablets of desired shape, thickness, hardness and disintegration.

### INHALATION FORMULATIONS

Sterile solutions for use in nebulizers are supplied in unit-dose, low-density polyethylene (LDPE) vials as a clear, colorless, sterile, preservative-free, aqueous solution containing different doses of R(-)-albuterol (0.63 mg, 1.25 mg, 5 mg, etc.). R(-)-albutero1,5.0 mg corresponds to R(-)-albuterol sulfate 6.0 mg. The concentrations shown here are examples only. Other concentrations may be manufactured to be used by the caring veterinary staff.

Metered dose dispensers may contain the API ( R-albuterol) as a solution or as a micronized suspension.

| Formula | Quantity contained in Each Metered Dose Dispenser 7.5 mL (10.5 g) Canister | |
|---|---|---|
| R-albuterol | 1.8 | mg |
| Trichloromonofluoromethane | 5.16 | g |
| Dichlorodifluoromethane | 5.16 | g |
| Sorbitan trioleate | 0.105 | g |

Each actuation delivers 90 mcg of R(-)-albuterol, corresponding to 108 microgram R(-)-albuterol sulfate. Multiple actuations will be given to the horse for acute treatment of airway obstruction. Metered dose dispensers may also dispense the API as a dry powder, as is well known to those skilled in the art.

### PARENTERAL FORMULATIONS

Sterile solutions for parenteral use are supplied in unit-dose, vials as a clear, colorless, sterile, preservative-free, aqueous solution in different strengths of R(-)-albuterol (1.25 mg, 5 mg, 20 mg, etc.). R(-)-albuterol 5.0 mg corresponds to R(-)-albuterol sulfate 6.0 mg. The doses shown here are examples only. Other vials with different doses of R(-)-albuterol solutions may be manufactured to be used by the veterinary staff for intravenous, subcutaneous or intramuscular injections or infusions.

### EQUIVALENTS

Those skilled in the art will recognize, or be able to ascertains using no more than routine experimentation, many equivalent to the specific embodiments of the invention described herein. Such equivalents, include the active isomers also of all other beta-adrenergic agonists than albuterol, such as for example clenbuterol, salmeterol, terbutaline, formoterol, fenoterol, ractopamine, hexoprenaline, isoprenaline, riniterol, isoetharine, metaproterenol, reproterenol, cimaterol, procaterol, carbuterol, tulobuterol, pibuterol, mabuterol, bitolterol, and bambuterol are intended to be encompassed in the scope of the following claims. In particular, the eutomeric isomers of salmeterol, formoterol and ractopamine are included in the present invention. Compounds with more than one chiral center have four or more isomers and one or more of said multiple isomers is/are therapeutically active. Those skilled in the art will realize that there are many pharmaceutically acceptable salt forms of the drugs of the inventions, such as for example**,** hydrochloride, sulfate, fumarate, hydrobromide, dihydrochloride, methanesulphonate, hydroxynaphthoate or where appropriate, one or other of the hydrate forms thereof, see Merck Index 11th edition (1989) items 9089, 209, 3927, 4628, 8223, 5053, 5836, 8142, 2347, 7765, 1840, 9720, 7461,1317, 4159, and 963 and references cited therein and, for Am. Rev. Resp. Dis. 1988,137: (4:2/2) 32.

Those skilled in the art will realize that routine preventive treatment of horses for heaves is not called for in healthy horses, but preventive treatment may be needed under special circumstances, such as for example prevention of exercise-induced bronchospasms.

SAD (small airways disease) in the horse is considered to be a form of heaves, where the smaller airways are blocked. The treatment of SAD is encompassed in the present invention. Treatments of other obstructive airway diseases or symptoms in the horse with R(-)-albuterol are also encompassed in the present invention.

Those skilled in the art will realize that repeated administration of an adrenergic beta-receptor agonist, including R(-)-albuterol, to horses will increase muscle mass, decrease body fat content and improve feed efficiency. The treatments of horses with R(-)-albuterol for one or more of such purposes are encompassed in the present invention.

Those skilled in the art will realize that R-albuterol can also be administered orally to horses, by administration of a food composition for horses, comprising the admixture with food materials, to horses suffering from heaves, of said optically active eutomer of albuterol.

## Claims

1. Use of the adrenergic beta-2 agonist R(-)-albuterol, or a pharmaceutically acceptable salt or solvate thereof for the manufacture of a medicament for treating heaves which minimizes or eliminates the side effects residing in the corresponding distomer.

2. The use of Claim 1, wherein the optical purity of R(-)-albuterol is:
(a) greater than 90% by weight; or
(b) than 99% by weight.

3. The use of Claim 1, wherein R(-)-albuterol is administered by injection, parenteral infusion, inhalation, transdermal administration or rectal administration or oral administration.

4. The use of Claim 1, wherein R(-)-albuterol is administered orally and the oral formulation consists of an instant release fraction of the dose and in addition thereto a delayed release fraction of the dose of R(-)-albuterol.

5. The use according to Claim 1, wherein either (a) the amount administered by inhalation is from about 1 microgram to about 200 micrograms per kilogram bodyweight, two to four times per day; or
(b) the amount administered orally is about 1 to about 200 mg, one to four times per day.

6. The use according to Claim 1, wherein R(-)-albuterol or a pharmaceutically acceptable salt thereof is administered together with:
(a) a pharmaceutically acceptable carrier;
(b) at least one compound that has anti-inflammatory activity; or
(c) at least one compound that has antibacterial activity.

7. The use according to Claim 1, wherein a therapeutic composition in the form of a tablet, capsule, injection or aerosol, which comprises a pharmaceutically acceptable carrier suitable for a tablet, capsule, injection or aerosol and an amount of R(-)-albuterol, or a pharmaceutically acceptable salt thereof, sufficient to alleviate symptoms of heaves, but insufficient to cause side effects, said R(-)-albuterol containing at least 99% by weight of R(-)-albuterol and less than 1% by weight of S-albuterol, is being administered to animals in need of such therapy and while avoiding the side effects residing in the distomeric isomer of the corresponding racemic mixture.

8. The use of claim 1, wherein said animal is a horse.

9. The use according to Claim 1, wherein a therapeutically effective amount of R(-)-albuterol or a pharmaceutically acceptable salt or solvate thereof is administered to prevent exercise-induced bronchospasm in horses.

10. The use of R(-)-albuterol, or a pharmaceutically acceptable salt or solvate thereof for the manufacture of a medicament for alleviating heaves symptoms in animals, wherein said R(-)-albuterol produces a therapeutic effect that is of longer duration than the corresponding effect of a comparable dose of racemic albuterol, and said R(-)-albuterol containing at least 98% by weight of R(-)-albuterol and less than 2% by weight of S(+)-albuterol.

## Patentansprüche

1. Verwendung des adrenergen beta-2 Agonisten R(-)-Albuterol oder eines pharmazeutisch annehmbaren Salzes oder Solvats davon zur Herstellung eines Medikaments zur Behandlung von Dämpfigkeit, welches die Nebenwirkungen minimiert oder eliminiert, die beim korrespondierenden Distomer bestehen.

2. Verwendung nach Anspruch 1, wobei die optische Reinheit von R(-)-Albuterol beträgt:
(a) größer als 90 Gew.-%; oder
(b) als 99 Gew.-%.

3. Verwendung nach Anspruch 1, wobei R(-)-Albuterol mittels Injektion, parenteraler Infusion, Inhalation, transdermaler Verabreichung oder rektaler Verabreichung oder oraler Verabreichung verabreicht wird.

4. Verwendung nach Anspruch 1, wobei R(-)-Albuterol oral verabreicht wird und die orale Formulierung aus einem Dosis-Anteil mit sofortiger Freisetzung besteht und zusätzlich dazu einem Dosis-Anteil mit verzögerter Freisetzung von R(-)-Albuterol.

5. Verwendung nach Anspruch 1, wobei entweder (a) die durch Inhalation verabreichte Menge zwischen etwa 1 Mikrogramm und etwa 200 Mikrogramm pro Kilogramm Körpergewicht liegt, zwei bis vier Mal pro Tag; oder (b) die oral verabreichte Menge etwa 1 bis etwa 200 Miligramm beträgt, ein bis viermal pro Tag.

6. Verwendung nach Anspruch 1, wobei R(-)-Albuterol oder ein pharmazeutisch annehmbares Salz davon zusammen verabreicht wird mit:
(a) einem pharmazeutisch annehmbaren Träger;
(b) wenigstens einer Verbindung, die entzündungshemmende Aktivität hat; oder
(c) wenigstens einer Verbindung, die antibakterielle Aktivität hat.

7. Verfahren nach Anspruch 1, wobei man eine therapeutische Zusammensetzung in Form einer Tablette, Kapsel, Injektion oder eines Aerosols, welche einen pharmazeutisch annehmbaren Träger aufweist geeignet für eine Tablette, Kapsel, Injektion oder ein Aerosol, und eine Menge R(-)-Albuterol, oder eines pharmazeutisch annehmbaren Salzes davon, die ausreicht, Symptome der Dämpfigkeit zu mildem, aber nicht ausreicht, Nebeneffekte zu bewirken, wobei R(-)-Albuterol wenigstens 99 Gew.-% R(-)-Albuterol und weniger als 1 Gew.-% S-Albuterol enthält, dem Tier verabreicht, welches solch eine Therapie benötigt, und während man die Nebenwirkungen, die beim distomeren Isomer der korrespondierenden racemischen Mischung besteht, vermeidet.

8. Verwendung nach Anspruch 1, wobei das Tier ein Pferd ist.

9. Verwendung nach Anspruch 1, wobei eine therapeutisch wirksame Menge R(-)-Albuterol oder eines pharmazeutisch annehmbaren Salzes oder Solvats davon verabreicht wird um trainingsinduzierten Bronchospasmen bei Pferden vorzubeugen.

10. Verwendung von R(-)-Albuterol oder eines pharmazeutisch annehmbaren Salzes oder Solvats davon zur Herstellung eines Medikaments zur Linderung von Dämpfigkeitssymptomen bei Tieren, wobei das R(-)-Albuterol eine therapeutische Wirkung produziert, welche von längerer Dauer ist als der entsprechende Effekt einer vergleichbaren Dosis racemischen Albuterols und wobei R(-)-Albuterol wenigstens 98 Gew.-% R(-)-Albuterol und weniger als 2 Gew.-% S(+)-Albuterol enthält.

## Revendications

1. Utilisation de l'agoniste bêta-2 adrénergique, R(-)-albuterol, ou d'un sel ou produit de solvatation pharmaceutiquement acceptable de celui-ci, pour la fabrication d'un médicament destiné à traiter la pousse qui minimise ou élimine les effets secondaires existant dans le distomère correspondant.

2. Utilisation selon la revendication 1, dans laquelle la pureté optique du R(-)-albuterol est :
(a) supérieure à 90 % en poids, ou
(b) supérieure à 99 % en poids.

3. Utilisation selon la revendication 1, dans laquelle le R(-)-albuterol est administré par injection, infusion parentérale, inhalation, administration transdermique ou administration rectale ou administration orale.

4. Utilisation selon la revendication 1, dans laquelle le R(-)-albuterol est administré par voie orale et la formulation orale est constituée d'une fraction à libération immédiate de la dose et, en plus de celle-ci, d'une fraction à libération retardée de la dose de R(-)-albuterol.

5. Utilisation selon la revendication 1, dans laquelle soit (a) la quantité administrée par inhalation est d'environ 1 microgramme à environ 200 microgrammes par kilogramme de poids corporel, deux à quatre fois par jours, soit (b) la quantité administrée par voie orale est d'environ 1 à environ 200 mg, une à quatre fois par jour.

6. Utilisation selon la revendication 1, dans laquelle le R(-)-albuterol ou un sel pharmaceutiquement acceptable de celui-ci est administré ensemble avec :
(a) un support pharmaceutiquement acceptable,
(b) au moins un composé qui a une activité anti-inflammatoire, ou
(c) au moins un composé qui a une activité antibactérienne.

7. Utilisation selon la revendication 1, dans laquelle une composition thérapeutique sous la forme d'une tablette, d'une capsule, d'une injection ou d'un aérosol, qui comporte un support pharmaceutiquement acceptable adapté à une tablette, une capsule, une injection ou un aérosol, et une quantité de R(-)-albuterol, ou d'un sel pharmaceutiquement acceptable de celui-ci, surf-fisante pour atténuer les symptômes de la pousse, mais insuffisante pour provoquer des effets secondaires, ledit R(-)-albuterol contenant au moins 99 % en poids de R(-)-albuterol et moins de 1 % en poids de S-albuterol, est administrée à des animaux ayant besoin d'une telle thérapie tout en évitant les effets secondaires existant dans l'isomère distomérique du mélange racémique correspondant.

8. Utilisation selon la revendication 1, dans laquelle ledit animal est un cheval.

9. Utilisation selon la revendication 1, dans laquelle une quantité thérapeutiquement efficace de R(-)-albuterol ou d'un sel ou produit de solvatation pharmaceutiquement acceptable de celui-ci, est administrée pour empêcher un bronchospasme induit par exercice chez des chevaux.

10. Utilisation de R(-)-albuterol, ou d'un sel ou produit de solvatation pharmaceutiquement acceptable de celui-ci pour la fabrication d'un médicament destiné à soulager des symptômes de la pousse chez des animaux, dans laquelle ledit R(-)-albuterol produit un effet thérapeutique qui n'est pas d'une durée plus longue que l'effet correspondant d'une dose comparable d'albuterol racémique, ledit R(-)-albuterol contenant au moins 98 % en poids de R(-)-albuterol et moins de 2 % en poids de S(+)-albuterol.
